# FASCICULE DE BREVET EUROPEEN

(11) **EP 2 723 261 B1**
(45) Date de publication et mention de la délivrance du brevet: **02.05.2018**
(21) Numéro de dépôt: 12734975.1
(22) Date de dépôt: 20.06.2012
(51) Int. Cl.: A61B 17/70

(54) **DISPOSITIF CHIRURGICAL POUR LA CORRECTION DE LA DEFORMATION DE LA COLONNE VERTEBRALE**
CHIRURGISCHE VORRICHTUNG ZUR KORREKTUR VON VERFORMUNGEN DER WIRBELSÄULE
SURGICAL DEVICE FOR CORRECTING DEFORMATIONS IN THE SPINAL COLUMN

(30) Priorité: 23.06.2011 FR 1155563
(43) Date de publication de la demande: 30.04.2014
(73) Titulaire: Spineway, 69130 Ecully (FR)
(72) Inventeur: LEROUX, Stéphane, F-69002 Lyon (FR); LAURITO, Philippe, F-83143 Le Val (FR); MARUENDA PAULINO, José Ignacio, E-46010 Valence (ES)
(74) Mandataire: Cabinet Laurent & Charras
(86) Numéro de dépôt international: PCT/FR2012/051392
(87) Numéro de publication internationale: WO 2012/175871

(56) Documents cités:
- US-A1- 2006 271 050
- US-A1- 2007 233 079
- US-A1- 2010 114 179

## Description

L'invention se rattache au secteur technique des instruments chirurgicaux pour la correction d'une déformation de la colonne vertébrale.

L'invention trouve une application particulièrement avantageuse pour la correction d'une scoliose.

L'invention concerne plus particulièrement le principe de base d'une correction telle que définie dans la demande de brevet FR1151331 dont le demandeur de la présente est également titulaire.

US 2006/0271050 décrit un dispositif chirurgical pour la correction de la déformation de la colonne vertébrale à l'aide de deux tiges engagées dans des tubes d'alignements couplés à des vis pédiculaires. L'alignement de la colonne vertébrale et de l'une des tiges se fait à l'aide de blocs insérés dans les tubes d'alignement qui permettent de conserver une géométrie prédéterminée de la colonne vertébrale. Pour l'essentiel, cette demande de brevet concerne un dispositif chirurgical pour la correction de la déformation de la colonne vertébrale. Ce dispositif comprend des éléments tubulaires aptes à être fixés temporairement au niveau de têtes de vis pédiculaires agencées pour être reliées par une tige cintrée implantable. Au moins une tige est destinée à être engagée, avec capacité de déplacement dans des lumières que présente chaque élément tubulaire, pour les aligner en vue de la correction de la dite colonne, par translation, bascule et rotation des vertèbres, dans les trois plans de l'espace de façon globale sur plusieurs vertèbres et directe par action sur la tige engagée dans lesdits éléments tubulaires. Chaque élément tubulaire présente des agencements pour être accouplés à l'extérieur des têtes de vis pédiculaires. La tige qui constitue une tige d'alignement et de manoeuvre, est apte à agir, au fur et à mesure de son déplacement, sur un moyen pour le déplacement concomitant de la tige cintrée, préalablement engagée dans les lumières, en direction des têtes de vis pédiculaires. Une autre tige est destinée à être accouplée à l'extérieur des éléments tubulaires disposés en alignement pour permettre le retrait de la tige d'alignement et de manoeuvre en vue de l'engagement, à l'intérieur de chaque élément tubulaire, d'un organe de manoeuvre d'un écrou de fixation de la tige cintrée dans chacune des têtes de vis pédiculaires.

De manière importante, dans ce dispositif chirurgical, chaque élément tubulaire est en deux parties indépendantes, l'une des extrémités de chaque partie possédant des agencements d'accouplement, d'une part, desdites parties entre elles, et d'autre part, par rapport à la tête de vis pédiculaire correspondante.

A partir de cette solution de base, un des problèmes que se propose de résoudre l'invention est d'améliorer la technique opératoire en facilitant le travail du chirurgien avec par ailleurs, la possibilité d'intervenir des deux côtés de la colonne (coté convexe et coté concave), avec toujours pour objectif d'assurer la double fonction, d'une part, de redresser les corps vertébraux au moyen des éléments tubulaires, et d'autre part, de positionner la tige implantable et la fixer au niveau des têtes de vis pédiculaires sans être obligé de démonter lesdits éléments tubulaires.

Un autre problème que se propose de résoudre également l'invention, est de pouvoir s'adapter au profil lordose/cyphose souhaité au final par le praticien, afin de répartir les efforts de distraction pour la mise en place de la tige cintrée implantable, simultanément sur de nombreuses vertèbres.

Pour résoudre un tel problème, le moyen de déplacement de la tige cintrée implantable est constituée par une bague montée avec capacité de coulissement le long de l'élément tubulaire, ladite bague étant agencée pour être réglable en hauteur afin de compenser les écarts dimensionnels entre la tige rectiligne d'alignement et de manoeuvre et la tige cintrée.

Il résulte de ces dispositions que la tige d'alignement et de manoeuvre, en combinaison avec les bagues réglables en hauteur, permettent d'exercer un effort d'appui sur la quasi-totalité de la surface de la tige cintrée.

Un autre problème que se propose de résoudre l'invention est de faciliter la descente des tiges dans les lumières des tubes en diminuant de manière significative l'effort qu'il est nécessaire d'exercer sur lesdites tiges. Pour résoudre un tel problème, l'extrémité de chaque élément tubulaire, à partir de laquelle est engagé l'organe de manoeuvre, présente une douille taraudée pour le vissage d'un organe de poussée agencé pour exercer un effort d'appui sur la tige d'alignement et de manoeuvre pour provoquer concomitamment le déplacement des bagues réglables en hauteur et de la tige cintrée implantable vers le bas.

Pour résoudre le problème posé d'exercer l'effort d'appui, l'organe de poussée présente une tige filetée apte à être vissée dans la douille taraudée, l'une des extrémités de ladite tige étant équipée d'une poignée de manoeuvre, tandis que l'autre extrémité est équipée d'un embout monté libre en rotation et présentant une échancrure de section complémentaire à celle de la tige d'alignement et de manoeuvre.

Pour résoudre le problème posé de pouvoir accoupler à l'extérieur des éléments tubulaires l'autre tige, après avoir retiré la tige d'alignement et de manoeuvre avec pour objectif de dégager le passage à l'intérieur des éléments tubulaires, l'extrémité de chaque élément tubulaire présente, à l'opposé de son accouplement avec la tête de vis pédiculaire, un collier rapporté avec une partie débordant latéralement pour l'engagement de l'autre tige.

Chaque collier peut se positionner plus ou moins haut le long des éléments tubulaires ou des bouchons. L'intérêt est de pouvoir adapter la position des instruments, notamment les organes espaceurs, à la morphologie du patient pour améliorer les corrections de la position des vertèbres.

Pour résoudre le problème posé de pouvoir accoupler les éléments tubulaires à l'extérieur de la tête de vis avec pour objectif de totalement libérer l'intérieur desdites têtes, chaque élément tubulaire est en deux parties indépendantes, l'une des extrémités de chaque partie présentant des agencements d'accouplement desdites parties entre elles et par rapport à la tête de vis pédiculaire correspondante.

Les agencements d'accouplement sont constitués par deux fourchettes aptes à être reliées avec capacité d'articulation pour permettre d'écarter les parties pour leur mise en place par rapport à la tête de vis pédiculaire puis les rabattre pour enserrer ladite tête maintenue entre lesdites parties par des moyens de retenue.

Pour résoudre le problème posé de constituer l'élément tubulaire en tant que tel pour l'engagement des différentes tiges, l'autre extrémité de chaque partie constituant ledit élément coopère, après avoir été rabattue, avec un écrou creux de liaison , lesdites parties délimitant les lumières diamétralement opposées pour l'engagement des tiges

L'invention est exposée ci-après plus en détail à l'aide des figures des dessins annexés dans lesquels :
- Les figures 1 et 2 sont des vues en perspective d'exemples de réalisation de bague avec capacité de réglage de leur hauteur.
- La figure 3 est une vue en perspective de l'organe de poussée

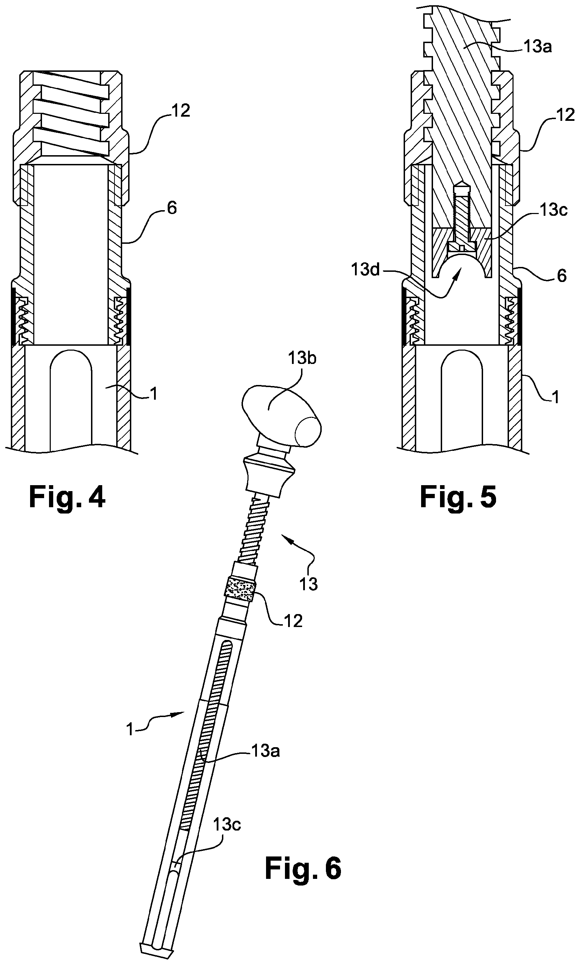

- La figure 4 est une vue partielle en coupe de l'extrémité supérieure d'un tube équipé d'une douille taraudée pour le vissage du corps de l'organe de poussée.
- La figure 5 est vue correspondant à la figure 4 montrant l'engagement de l'extrémité du corps de l'extracteur.
- La figure 6 est une vue en perspective montrant la mise en place de l'organe de poussée dans un élément tubulaire.
- Les figures 7 à 19 sont des vues en perspective montrant les principales étapes de la correction de la colonne vertébrale au moyen du dispositif chirurgical selon l'invention.

Le dispositif chirurgical selon l'invention constitue des améliorations techniques au dispositif décrit dans la demande de brevet précité FR 115133.

A cet égard, on rappelle pour une meilleure compréhension de la suite de la description, que le dispositif chirurgical comprend des éléments tubulaires (1) aptes à être fixés temporairement au niveau des têtes de vis pédiculaires (2) agencées pour être reliées par une tige cintrée implantable (3) de distraction ou de compression, en vue d'assurer la correction directement et globalement simultanément sur plusieurs vertèbres, dix par exemple. Une tige (4) est destinée à être engagée au travers de lumières (1c) et (1d) que présentent les différents éléments tubulaires (1). La tige (4) constitue une tige d'alignement et de manoeuvre pour aligner les éléments tubulaires (1), en vue d'assurer la correction de la colonne vertébrale suite à une rotation, un décalage et une bascule, ceci dans les trois plans de l'espace, de plusieurs vertèbres (V) résultant dudit alignement.

Le but recherché, est de pouvoir aligner les différents éléments tubulaires (1) conduisant au redressement des différentes vertèbres (V), puis, dans le même temps opératoire, sans aucun démontage, mettre en place la tige cintrée de correction implantable (3) dans les différentes têtes de vis (2) afin de fixer la correction obtenue. A noter qu'il n'y a pas de perte de la correction obtenue, l'insertion de la tige cintrée se produit au centre des éléments tubulaires de guidage, lorsque la correction est obtenue.

Chaque élément tubulaire (1) présente des agencements pour être accouplé à l'extérieur des têtes de vis pédiculaires (2).

A noter que la forme de la partie inférieure de chaque élément tubulaire possède une très large surface de contact avec les faces extérieures de la tête de vis, ce qui permet une importante solidarité et cohésion entre les éléments tubulaires et les vis, afin de déplacer, corriger et contrôler le déplacement de la position des vertèbres pour rectifier la déformation.

Chaque élément tubulaire (1) est en deux parties indépendantes (1a) et (1b). L'une des extrémités de chaque partie (1a) et (1b) présente des agencements conformés pour assurer l'accouplement, d'une part, des parties entre elles et, d'autre part, par rapport à la tête de vis pédiculaire correspondante (2). Ces agencements d'accouplement sont, par exemple, constitués par deux fourchettes aptes à être reliées avec capacité d'articulation. Par exemple, les branches de l'une des fourchettes présente intérieurement des ergots faisant office d'axe coopérant avec des échancrures que présente la face externe des branches de l'autre fourchette.

L'accouplement des deux parties (1a) et (1b), qui constituent donc des éléments demi-tubulaires, est réalisé de façon circulaire au contact de la face externe des têtes de vis pédiculaires (2).

Ces dispositions permettent d'écarter les parties (1a) et (1b) pour leur mise en place par rapport à la tête de vis pédiculaire (2), puis de les rabattre pour enserrer ladite tête de vis pédiculaire. A noter que la tête de vis pédiculaire (2) présente des agencements de retenue coopérant avec des agencements complémentaires d'au moins l'une des parties (1a) et (1b). Par exemple, ces agencements sont constitués par au moins un pion ou autre moyen d'indexation que présente l'une des parties (1a) et (1b) à proximité de la fourchette correspondante et apte à coopérer avec un trou borgne que présente une partie de la tête de vis pédiculaire (2).

Après accouplement des parties (1a) et (1b), comme indiqué, ces dernières délimitent les lumières diamétralement opposées (1c) et (1d).

La tige d'alignement (4) est engagée dans les lumières (1c)et (1d) avec capacité de déplacement perpendiculaire à l'axe des éléments tubulaires (1).

L'autre extrémité de chaque partie (1a) et (1b) coopère, après avoir été rabattue comme indiqué précédemment, avec un bouchon creux (6) apte à assurer la liaison desdites parties pour constituer l'élément tubulaire en tant que tel.

Comme il sera indiqué dans la suite de la description, la tige (4) est apte à agir, au fur et à mesure de son déplacement, sur un moyen (7) pour le déplacement concomitant de la tige cintrée implantable (3) préalablement engagée dans les lumières (1c) et (1d).

On rappelle que ces différentes caractéristiques ressortent pour l'essentiel de l'enseignement du document FR 1151331.

Selon la présente invention, le moyen de déplacement (7) de la tige cintrée implantable (3) est constituée par une bague montée avec capacité de coulissement le long de l'élément tubulaire. La bague (7) est agencée pour être réglable en hauteur afin de compenser les écarts dimensionnels entre la tige rectiligne (4) et la tige cintrée implantable (3). Avantageusement, chaque bague (7) est en deux parties (7a) et (7b) accouplées entre elles avec capacité de réglage pour faire varier sa hauteur.

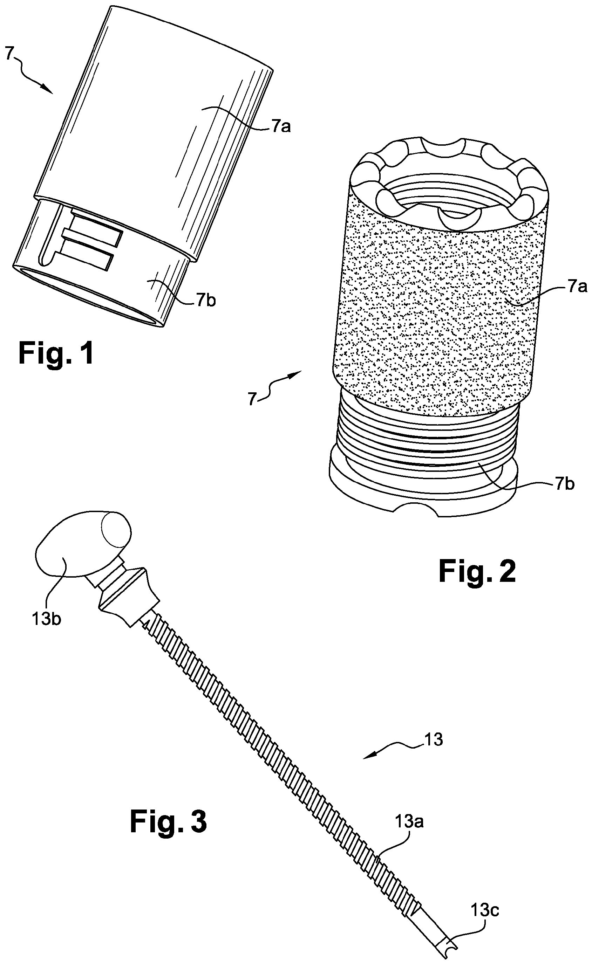

Dans la forme de réalisation illustrée figure 2, les deux parties (7a) et (7b) sont accouplées par un système de filetage spécifique ou bien les parties (7a) et (7b) sont montées avec capacité d'indexation angulaire dans la position souhaitée (figure 1). La bague (7) peut présenter à sa base des échancrures diamètralement opposées aptes à se positionner en regard des lumières (1c) et (1d) pour coopérer avec la section de la tige cintrée (3). Ces dispositions assurent l'indexation de la tige cintrée, lui évitant de tourner sur elle-même au fur et à mesure de son déplacement comme il sera indiqué dans la suite de la description. Le règlage en hauteur des bagues (7) permet de répartir les efforts tout le long de la tige (3). A noter qu'au départ de l'intervention les bagues (7) sont à une hauteur minimum permettant d'insérer la tige (3) le plus près possible de la tige d'alignement et de manoeuvre (4).

A titre indicatif, la hauteur des bagues (7), peut varier entre 15 et 50mm environ. A noter également que la bague (7) peut présenter des agencements permettant de la maintenir temporairement en hauteur, sur les tubes tant qu'un effort d'appui n'est pas exercé sur ces dernières.

Suivant une autre caractéristique, l'extrémité de chaque élément tubulaire (1) considérée à l'opposé de l'extrémité d'accouplement avec les têtes de vis pédiculaires, présente une douille taraudée (12) vissée par exemple sur les bouchons (6). Dans cette douille taraudée (12) est vissé un organe de poussée (13) agencé pour exercer un effort d'appui sur la tige d'alignement et de manoeuvre (4) afin de provoquer concomitamment le déplacement des bagues (7) et de la tige cintrée (3). Le corps de l'organe de poussée (13) est constitué par une tige filetée (13a) dont la longueur est sensiblement égale à celle des éléments tubulaires (1). L'une des extrémités de cette tige filetée (13a) est équipée d'une poignée de manoeuvre (13b) tandis que son autre extrémité est équipée d'un embout (13c). Cet embout (13c) est monté libre en rotation, en bout de la tige filetée (13a) et présente une échancrure (13d) complémentaire à celle de la tige d'alignement et de manoeuvre (4).

Il résulte donc de ces dispositions que lorsque la tige filetée (13a) est vissée dans la douille (12), cette dernière est enfoncée verticalement du haut vers le bas dans l'élément tubulaire (1) au fur et à mesure de son vissage pour exercer un appui sur la tige d'alignement et de manoeuvre (4) par l'intermédiaire de l'embout (13c) lequel n'est pas entrainé en rotation, en demeurant indexé par rapport à ladite tige (13a).

Il résulte, donc de ces dispositions, qu'en exerçant une action de vissage par l'organe de poussée (13), on provoque le déplacement de l'embout (13d) qui prend appui sur la tige (4), pour assurer, au fur et à mesure de l'action de vissage, le déplacement des différentes bagues (7) dont les hauteurs ont été préalablement réglées pour compenser les écarts dimensionnels entre ladite tige (4) qui est rectiligne, et la tige implantable (3) qui est cintrée. Le déplacement des bagues (7), en appui sur la tige (3), provoque le déplacement sur cette dernière pour la positionner dans les vis pédiculaires correspondantes.

Ce positionnement est possible étant donné que les têtes de vis (2) sont totalement dégagées suite à l'accouplement et l'alignement des éléments tubulaires (1) qui s'effectue par l'extérieur desdites têtes de vis pédiculaires. Par exemple, au moment de l'intervention les organes de poussée (13) sont montés respectivement sensiblement aux extrémités et au milieu de la série d'éléments tubulaires considérés.

On rappelle également que l'extrémité de chaque élément tubulaire (1) considéré à l'opposé de l'extrémité coopérant avec les têtes de vis pédiculaires, reçoit un collier rapporté (8) présentant une partie (8a) débordant latéralement pour l'engagement d'une tige (5) destinée à maintenir le réglage obtenu après avoir retiré la tige (4).

On rappelle que les différents éléments tubulaires (1) sont reliés, deux à deux, au niveau de leur extrémité libre, par un organe espaceur (9).

Le positionnement variable des colliers (8) permet d'adapter les organes espaceurs par exemple, à la morphologie du patient.

La longueur de cet organe espaceur (9) est variable, au choix du chirurgien ; cette longueur détermine la distance entre les éléments tubulaires qui conduit à déplacer les vertèbres entre elles. Ceci permet au chirurgien de contrôler la courbure de la section de colonne implantée, et par là-même de restaurer une courbure physiologique (par exemple cyphose thoracique).

Concernant les organes espaceurs, il est proposé une gamme de longueurs différentes.

Chaque organe espaceur permet de solidariser deux éléments tubulaires contigus dont la position forme un angle entre eux. Chaque organe espaceur est constitué d'une plaque présentant deux trous ronds munis chacun d'un anneau formant une rotule mobile, inséré dans l'épaisseur de la plaque. Le maintien intervient lorsqu'on glisse l'anneau le long de chaque élément tubulaire.

Chaque tête de vis pédiculaire (2) est constituée par une douille avec une échancrure (2a) pour l'engagement de la tige cintrée implantable (3). Selon l'invention, la tête de vis pédiculaire (2) est prolongée, de part et d'autre de l'échancrure (2a), par des pattes sécables (2b) et (2c). De la même façon que l'alésage de la douille, les pattes (2b) et (2c) sont taraudées pour la mise en place d'un écrou (10) en vue de la fixation de la tige (3) dans la tête de vis pédiculaire correspondante. L'écrou (10) est mis en place par un organe de manoeuvre (10), engagé dans les éléments tubulaires (1), après avoir retiré la tige (4) et positionné la tige (5) dans les colliers (8), à l'extérieur des éléments tubulaires (1).

Compte tenu des caractéristiques à la base du dispositif de correction selon l'invention, la méthode est décrite ci-après en référence aux figures è à 19.

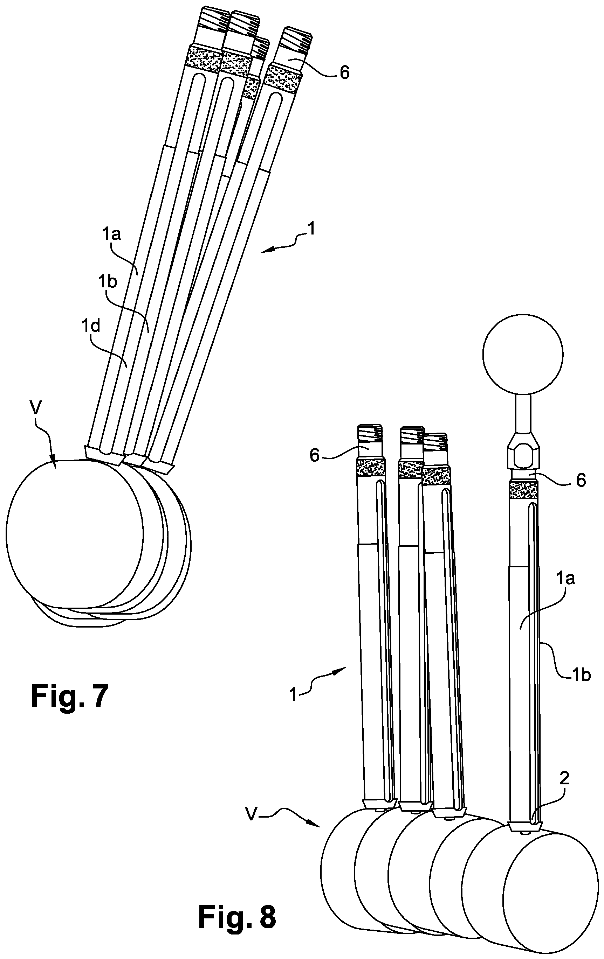

On accouple, sur chacune des têtes de vis (2), les éléments tubulaires (1), dans les conditions indiquées précédemment, c'est-à-dire sur l'extérieur des têtes de vis, pour dégager l'intérieur desdites têtes de vis (figure 7).

Les différentes vis pédiculaires à ailettes (2), équipées des éléments tubulaires (1), sont fixées dans les corps vertébraux (V) de chaque côté. A noter que les têtes de vis pédiculaires peuvent être du type monobloc ou du type polyaxial, comme cela est parfaitement connu pour un homme du métier.

Chaque élément tubulaire (1) est ensuite équipé de son bouchon (écrou de fixation) (6) (figure 7). De même, il est possible d'utiliser une poignée ronde pour se servir de l'élément tubulaire comme d'un tournevis ; cette poignée coopérant avec les bouchons (figure 8). La bague (7) est positionnée en partie haute de l'élément tubulaire considéré. A ce stade, les éléments tubulaires (1) sont décalés angulairement en correspondance avec la déformation de la colonne vertébrale.

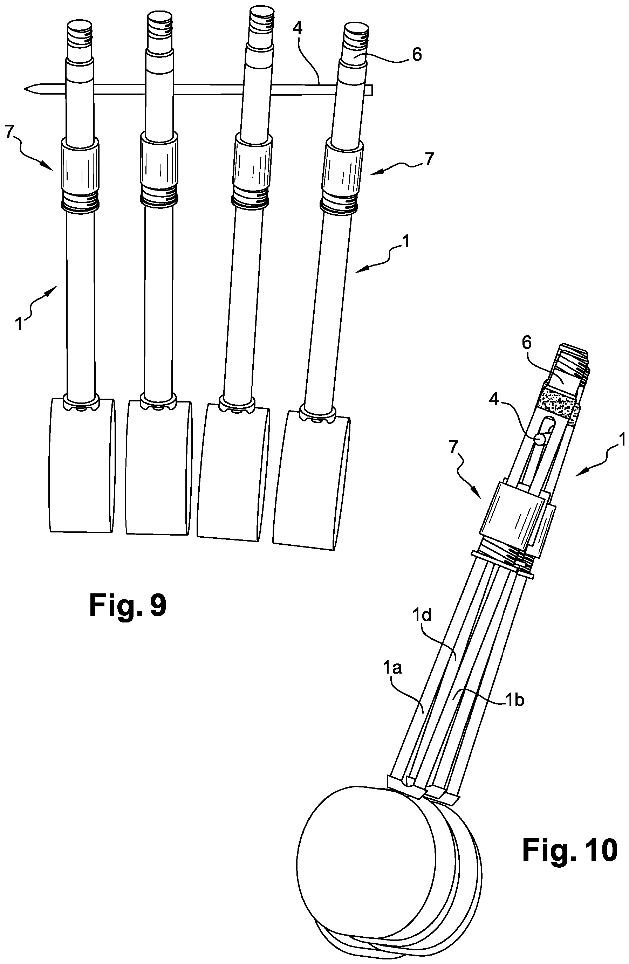

On engage ensuite la tige (4) au travers des lumières (1c) et (1d) des différents éléments tubulaires (1) et au-dessus des bagues (7) pour provoquer leur redressement progressif en vue de leur alignement en générant simultanément une translation, une bascule et une rotation des vertèbres dans les trois plans de l'espace pour la correction de la colonne (figures 9 et 10).

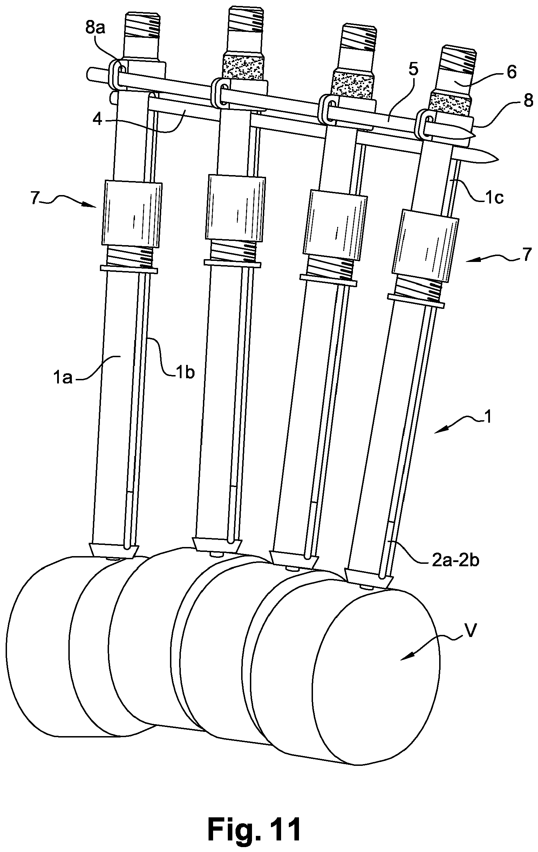

Les colliers (8) sont également disposés en partie haute des éléments tubulaires (1) pour l'engagement de la tige (5) (figure 11).

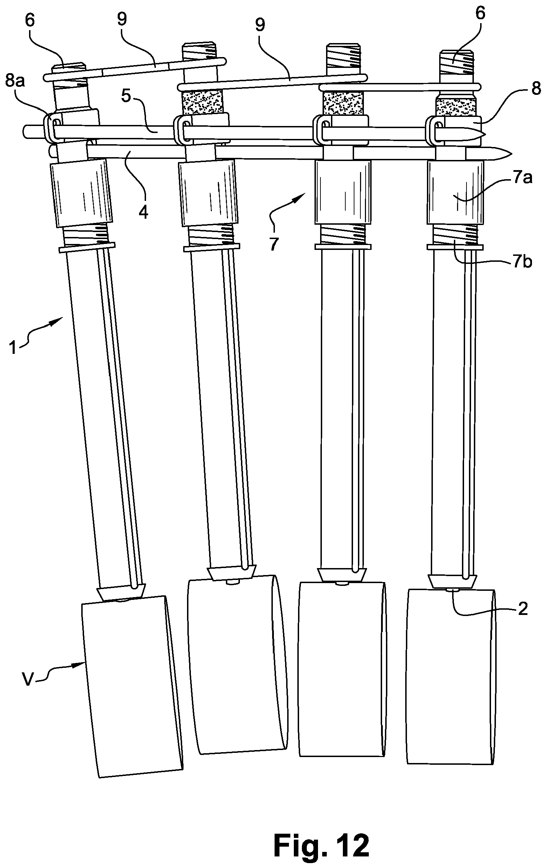

Les différents éléments tubulaires (1) sont ensuite accouplés, deux à deux, par les organes espaceurs (9) (figure 12), afin de prédéterminer la correction de courbure attendue, telle qu'une cyphose thoracique par exemple.

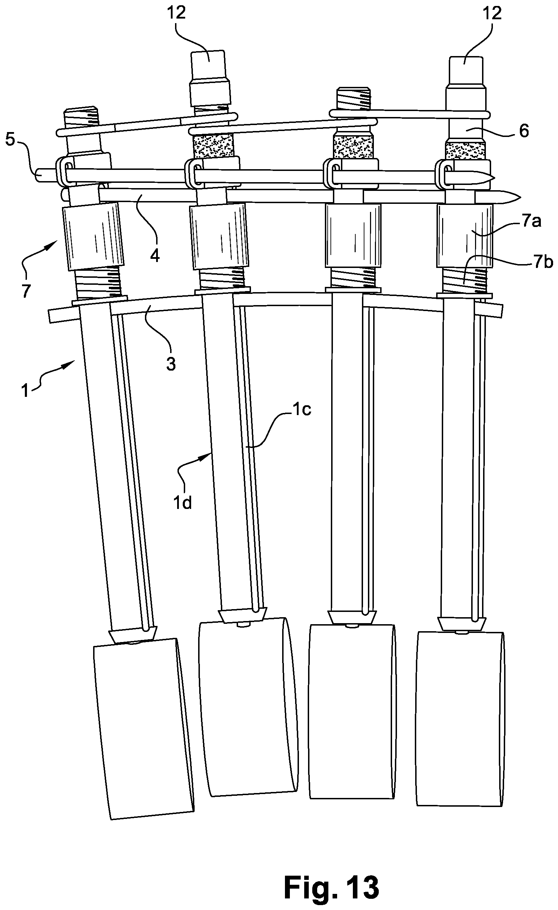

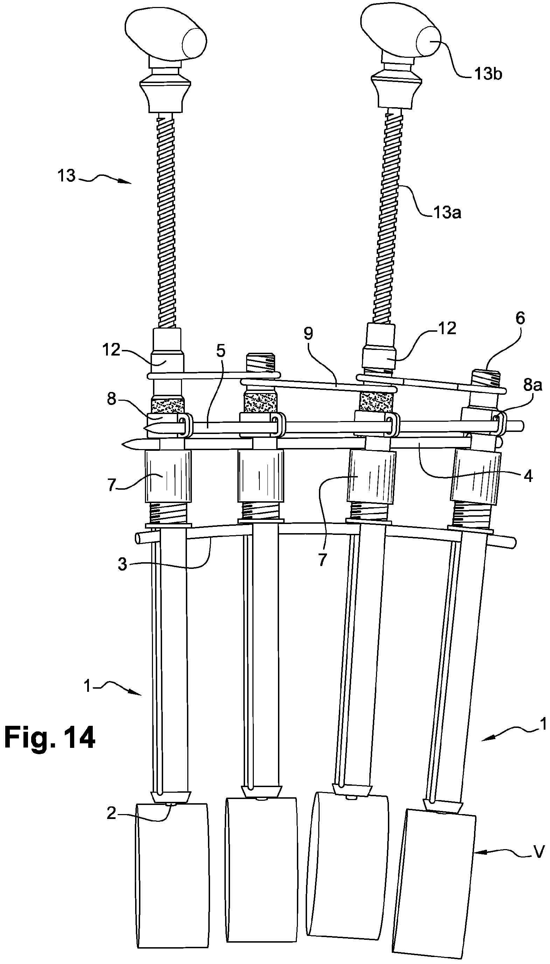

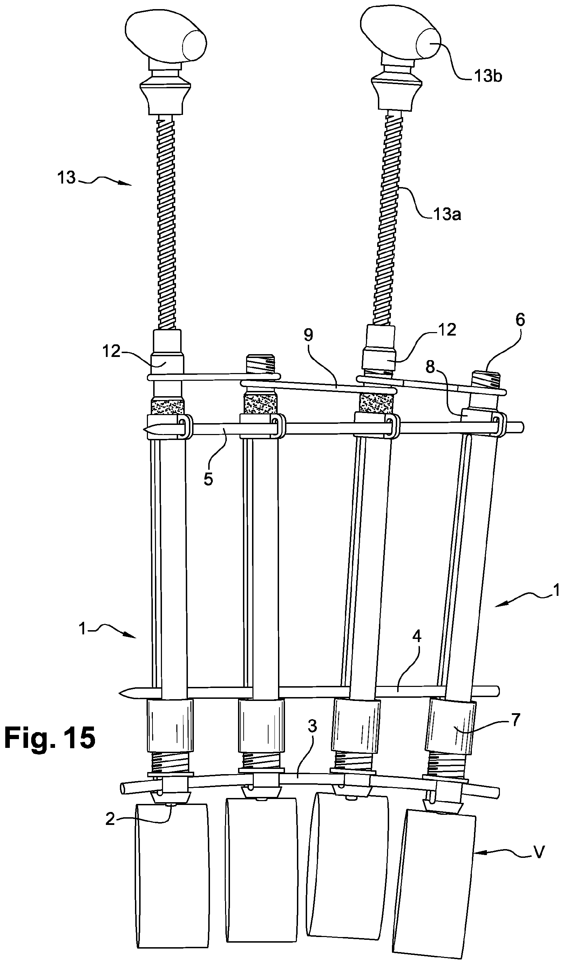

On engage transversalement, sous les bagues (7) (figure 13), la tige cintrée implantable (3) en vue de son positionnement et sa fixation dans les têtes de vis pédiculaires (2a). Dans ce but, on équipe les extrémités libres de certains éléments tubulaires (1), de la douille taraudée (12), pour le vissage de 1' organe de poussée (13) dont l'embout (13d) vient en appui sur la tige (4) (figure 14). L'action de vissage de l'organe de poussée (13) provoque le déplacement des différentes bagues (7) et le déplacement concomitant de la tige cintrée (3) jusqu'à ce que cette dernière vienne se positionner dans les échancrures des douilles des têtes de vis pédiculaires (figure 15).

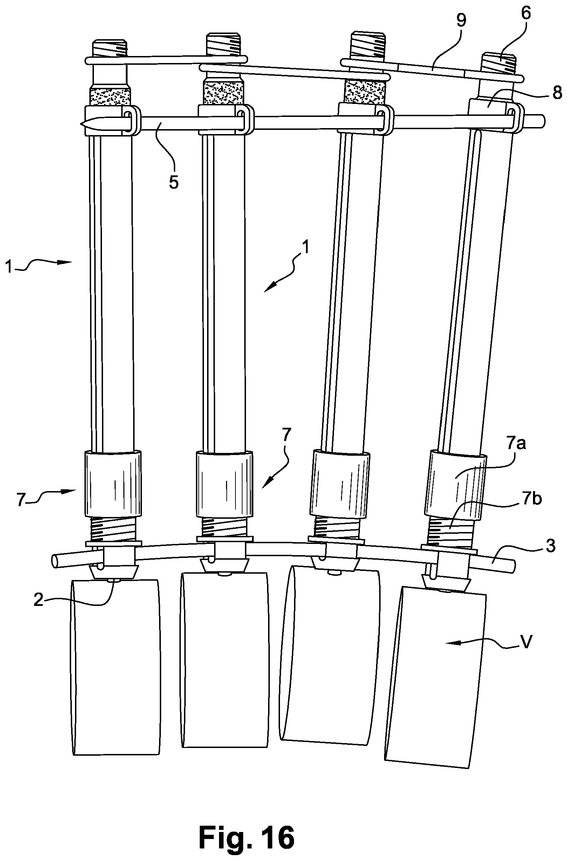

On retire ensuite les organes de poussée (13) et la tige de manoeuvre (4) (figure 16).

A noter que l'ensemble des éléments tubulaires (1) alignés permet, grâce à une tige de longueur choisie et variable, de mobiliser un groupe déterminé de vertèbres, et de lui appliquer un déplacement visant à entrainer les côtes reliées aux vertèbres. Ceci permet de corriger les déformations du grill costal composant la gibbosité des patients scoliotiques. Cette mobilisation d'un groupe limité de vertèbres et des côtes qui y sont reliées, peut s'effectuer après avoir réduit dans les trois plans de l'espace et de façon globale la position de l'ensemble des vertèbres.

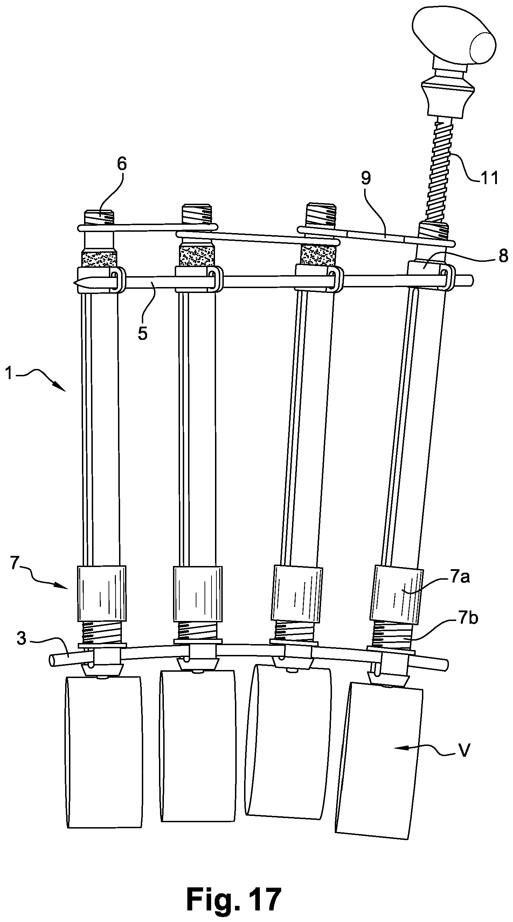

L'intérieur des différents éléments tubulaires (1) et des têtes de vis pédiculaires (2), étant totalement libre, il est, par conséquent, possible d'introduire des écrous (10) au travers des différents éléments tubulaires (1) par l'organe de manoeuvre approprié (11) (figure 17) et de visser les écrous (10) dans les têtes de vis pédiculaires pour assurer la fixation de la tige implantable (3).

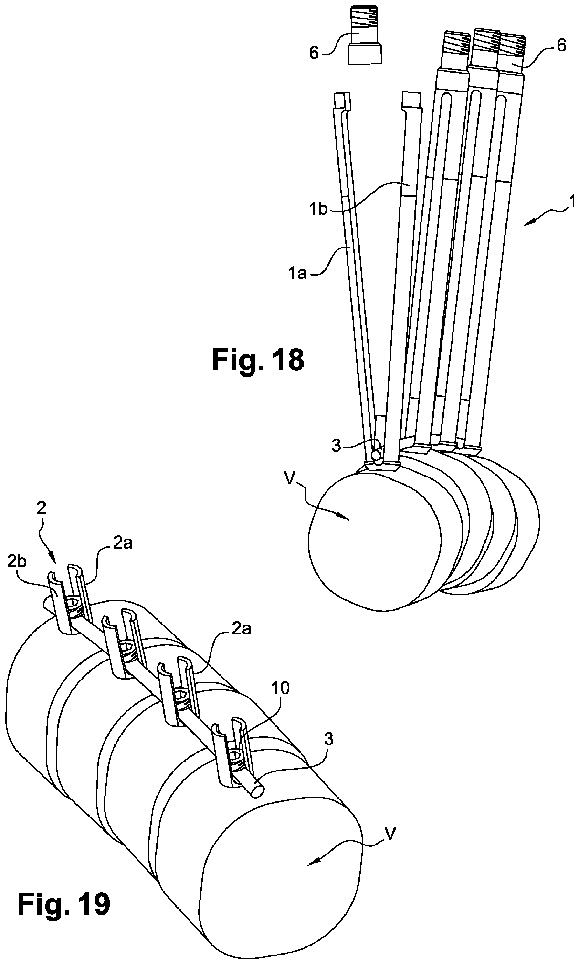

A ce stade du mode opératoire, il est alors possible de démonter le dispositif et, par conséquent, de retirer les éléments tubulaires (figure 18).

Il suffit ensuite d'assurer le vissage complet des écrous (10) au moyen d'un organe pour assurer la liaison et la fixation définitive de la tige cintrée (3) dans les têtes de vis pédiculaires (2), de casser les parties sécables (2b) et (2c) des vis pédiculaires (2) (figure 19).

Les avantages ressortent bien de la description, en particulier on souligne et on rappelle en plus de ceux relevant du document FR 1151331 :
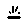 Le réglage en hauteur des bagues d'appui pour compenser les écarts dimensionnels correspondant au profil lordose/cyphose.
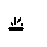 L'utilisation d'un organe de poussée pour faciliter la descente de la tige implantable, avec un minimum d'efforts.

## Revendications

1. Dispositif chirurgical pour la correction de la déformation de la colonne vertébrale comprenant des éléments tubulaires (1) aptes à être fixés temporairement au niveau de têtes de vis pédiculaires agencées pour être reliées par une tige cintrée implantable (3), au moins une tige (4) destinée à être engagée, avec capacité de déplacement dans des lumières (1c) et (1d) que présente chaque élément tubulaire, pour les aligner correspondant à la correction de la dite colonne, par translation, bascule et rotation des vertèbres dans les trois plans de l'espace, chaque élément tubulaire (1) présente des agencements pour être accouplés à l'extérieur des têtes de vis pédiculaires (2), la tige (4) qui constitue une tige d'alignement et de manoeuvre, étant apte à agir, au fur et à mesure de son déplacement, sur un moyen (7) pour le déplacement concomitant de la tige cintrée (3), préalablement engagée dans les lumières (1c) et (1d), en direction des têtes de vis pédiculaires (2), une autre tige (5) étant destinée à être accouplée à l'extérieur des éléments tubulaires (1) disposés en alignement pour permettre le retrait de la tige d'alignement et de manoeuvre (4) en vue de l'engagement, à l'intérieur de chaque élément tubulaire, d'un organe de manoeuvre (11) d'un écrou (10) de fixation de la tige cintrée dans chacune des têtes de vis pédiculaires (2),
**caractérisé en ce que** le moyen (7) de déplacement de la tige cintrée implantable (3) est constitué par une bague montée avec capacité de coulissement le long de l'élément tubulaire (1), ladite bague étant en deux parties (7a) et (7b) accouplées entre elles avec capacité de réglage pour faire varier sa hauteur afin de compenser les écarts dimensionnels entre la tige rectiligne d'alignement et de manoeuvre (4) et la tige cintrée (3).

2. Dispositif selon la revendication 1, **caractérisé en ce que** l'extrémité de chaque élément tubulaire (1), à partir de laquelle est engagé l'organe de manoeuvre, présente une douille taraudée (12) pour le vissage d'un organe de poussée (13) agencé pour exercer un effort d'appui sur la tige d'alignement et de manoeuvre (4) pour provoquer concomitamment le déplacement des bagues (7) et de la tige cintrée (3).

3. Dispositif selon la revendication 2, **caractérisé en ce que** l'organe de poussée (13) présente une tige filetée (13a) apte à être vissée dans le douille taraudée (12), l'une des extrémités de ladite tige (13a) étant équipée d'une poignée de manoeuvre (13b), tandis que l'autre extrémité est équipée d'un embout (13c) monté libre en rotation et présentant une échancrure (13d) de section complémentaire à celle de la tige d'alignement et de manoeuvre (4).

4. Dispositif selon l'une quelconque des revendications 1-3, **caractérisé en ce que** l'extrémité de chaque élément tubulaire (1) présente, à l'opposé de son accouplement avec la tête de vis pédiculaire, un collier rapporté (8) avec une partie (8a) débordant latéralement pour l'engagement de l'autre tige (5), dont le positionnement est variable en hauteur.

5. Dispositif selon l'une quelconque des revendications 1- 4, **caractérisé en ce que** chaque élément tubulaire (1) est en deux parties indépendantes (1a) et (1b), l'une des extrémités de chaque partie présentant des agencements d'accouplement desdites parties entre elles et par rapport à la tête de vis pédiculaire correspondante (2).

6. Dispositif selon la revendication 5, **caractérisé en ce que** les agencements d'accouplement sont constitués par deux fourchettes aptes à être reliées avec capacité d'articulation pour permettre d'écarter les parties (1a) et (1b) pour leur mise en place par rapport à la tête de vis pédiculaire (2) puis les rabattre pour enserrer ladite tête maintenue entre lesdites parties par des moyens de retenue.

7. Dispositif selon l'une quelconque des revendications 1, 5 et 6, **caractérisé en ce que** l'autre extrémité de chaque partie (1a) et (1b) coopère, après avoir été rabattue, avec un écrou creux de liaison (6) pour constituer l'élément tubulaire en tant que tel, lesdites parties (1a) et (1b) délimitant les lumières diamétralement opposées (1c) et (1d).

8. Dispositif selon la revendication 1 **caractérisé en ce qu'**une tige de manoeuvre de longueur variable relie un groupe d'éléments tubulaires sélectionnés après correction et alignement dans les trois plans de l'espace, afin d'assurer une mobilisation sélective permettant de réduire une gibbosité.

## Patentansprüche

1. Chirurgisches Gerät zur Korrektur von Verformungen der Wirbelsäule, umfassend rohrförmige Elemente (1), die vorübergehend an pedikulären Schraubenköpfen befestigt werden können, die so angeordnet sind, dass sie über eine implantierbare gebogene Stange (3) verbunden werden können, mindestens eine Stange (4), die dazu bestimmt ist, eingerastet zu werden, wobei sie in den Öffnungen (1c) und (1d), die jedes rohrförmige Element aufweist, verschoben werden kann, um sie entsprechend der Korrektur dieser Wirbelsäule durch Translation, Kippen und Rotation der Wirbel in den drei Raumebenen auszurichten, wobei jedes rohrförmige Element (1) Vorrichtungen aufweist, mit denen es außerhalb der pedikulären Schraubenköpfe (2) angekuppelt werden kann, wobei die Stange (4), die eine Ausrichtungs- und Bedienstange bildet, in der Lage ist, entsprechend ihrer Verschiebung auf eine Vorrichtung (7) zur entsprechenden Verschiebung der gebogenen Stange (3) einzuwirken, die zuvor in die Öffnungen (1c) und (1d) in Richtung der pedikulären Schraubenköpfe (2), eingerastet wurde, wobei eine weitere Stange (5) dazu bestimmt ist, außen an die rohrförmigen Elemente (1) gekoppelt zu werden, die fluchtend angeordnet sind, so dass die Ausrichtungs- und Bedienstange (4) im Hinblick auf das Einrasten, im Innern jedes rohrförmigen Elementes, eines Bedienorgans (11) einer Befestigungsmutter (10) der gebogenen Stange in jedem der pedikulären Schraubenköpfe (2) herausgenommen werden kann, ,
**dadurch gekennzeichnet, dass** die Vorrichtung (7) zur Verschiebung der implantierbaren gebogenen Stange (3) aus einem Ring besteht, der entlang rohrförmiger Elemente (1) verschiebbar montiert ist, wobei dieser Ring aus zwei Teilen (7a) und (7b) besteht, die miteinander einstellbar verkuppelt sind, um die Höhe zu variieren, um Maßabweichungen zwischen der geraden Ausrichtungs-und Bedienstange (4) und der gebogenen Stange (3) auszugleichen.

2. Gerät nach Anspruch 1, **dadurch gekennzeichnet, dass** das Endstück jedes der rohrförmigen Elemente (1), von denen ausgehend das Bedienorgang eingerastet wird, eine Gewindehülse (12) zum Anschrauben eines Druckelementes (13) aufweist, die dazu vorgesehen ist, eine Druckkraft auf die Ausrichtungs- und Bedienstange (4) auszuüben, um dadurch die Verschiebung der Ringe (7) und der gebogenen Stange (3) zu veranlassen.

3. Gerät nach Anspruch 2, **dadurch gekennzeichnet, dass** das Druckelement (13) eine Gewindestange (13a) aufweist, die in die Gewindehülse (12) eingeschraubt werden kann, wobei eines der Endstücke dieser Stange (13a) mit einem Bediengriff (13b) ausgestattet ist, während das andere Endstück mit einem frei drehbaren Stutzen (13c) ausgestattet ist, der eine Einbuchtung (13d) aufweist, deren Querschnitt komplementär zu dem der Ausrichtungs- und Bedienstange (4) ist.

4. Gerät nach einem der Ansprüche 1-3, **dadurch gekennzeichnet, dass** das Endstück jedes rohrförmigen Elementes (1) auf der zu seiner Verkupplung mit dem pedikulären Schraubenkopf entgegengesetzten Seite eine aufgesetzte Schelle (8) aufweist, bei der ein Teil (8a) seitlich überragt, damit die andere Stange (5), deren Position in der Höhe variabel ist, einrasten kann.

5. Gerät nach einem der Ansprüche 1-4, **dadurch gekennzeichnet, dass** jedes rohrförmige Element (1) aus zwei unabhängigen Teilen (1a) und (1b) besteht, wobei eines der Endstücke Vorrichtungen zur Verkupplung dieser Teile miteinander und bezogen auf den entsprechenden pedikulären Schraubenkopf (2) aufweist.

6. Gerät nach Anspruch 5, **dadurch gekennzeichnet, dass** die Kupplungsvorrichtungen aus zwei Gabeln bestehen, die miteinander verbunden werden können, die voneinander wegbewegt werden können, um die Teile (1a) und (1b) zwecks Positionierung bezogen auf den pedikulären Schraubenkopf (2) voneinander abzuspreizen und sie dann zurückzuklappen, um diesen Kopf einzuspannen, der zwischen diesen Teilen durch Haltevorrichtungen gehalten wird.

7. Gerät nach einem der Ansprüche 1, 5 und 6, **dadurch gekennzeichnet, dass** das andere Endstück jedes Teils (1a) und (1b), nachdem es zurückgeklappt wurde, mit einer Verbindungs-Hohlmutter (6) zusammenarbeitet, um das rohrförmige Element als solches zu bilden, wobei die Teile (1a) und (1b) die diametral entgegengesetzten Öffnungen (1c) und (1d) begrenzen.

8. Gerät nach Anspruch 1, **dadurch gekennzeichnet, dass** eine Bedienstange variabler Länge eine Gruppe von rohrförmigen Elementen verbindet, die nach Korrektur und Ausrichtung in den drei Ebenen des Raums gewählt werden, um eine selektive Mobilisierung sicherzustellen, mit der eine Gibbusbildung verringert werden kann.

## Claims

1. A surgical device for correcting deformations in the spinal column comprising tubular elements (1) capable of being temporarily attached at the level of pedicle screw heads arranged to be connected by an implantable curved rod (3), at least one rod (4) intended to be movably inserted into slots (1c) and (1d) of each tubular element to align them for the correction of said column by translation, tilting, and rotation of the vertebrae in the three planes of space, each tubular element (1) being designed in such a way that it can be coupled to the outside of the pedicle screw heads (2), the rod (4) which forms an alignment and handling rod being capable of acting, as it moves, on means (7) for simultaneously displacing the curved rod (3), previously inserted into the slots (1c) and (1d) towards the pedicle screw heads (2), another rod (5) being intended to be coupled to the outside of the aligned tubular elements (1) to enable the removal of the alignment and handling rod (4) in order to insert into each tubular element a member (11) for actuating a nut (10) for fastening the curved rod in each of the pedicle screw heads (2),
**characterized in that** the means (7) for displacing the implantable curved rod (3) are formed of a ring assembled with the ability to slide along the tubular element (1), said ring being made of two parts (7a) and (7b) coupled together in height-adjustable fashion to compensate for dimensional deviations between the rectilinear alignment and handling rod (4) and the curved rod (3).

2. The device of claim 1, **characterized in that** the end of each tubular element (1), from which the actuation member is engaged, comprises a tapped socket (12) for the screwing of a pusher member (13) arranged to press on the alignment and handling rod (4) to simultaneously cause the displacement of the rings (7) and of the curved rod (3).

3. The device of claim 2, **characterized in that** the pusher member (13) comprises a threaded rod (13a) capable of being screwed into the tapped socket (12), one of the ends of said rod (13a) being fitted with a control handle (13b), while the other end is fitted with a rotatably assembled tip (13c) having a recess (13d) with a section complementary to that of the alignment and handling rod (4).

4. The device of any of claims 1-3, **characterized in that** the end of each tubular element (1) has, opposite to its coupling with the pedicle screw head, an added collar (8) with a laterally-protruding portion (8a) for the insertion of the other height-adjustable rod (5).

5. The device of any of claims 1-4, **characterized in that** each tubular element (1) is made of two independent parts (1a) and (1b), one of the ends of each part having fittings for coupling said parts together and with the corresponding pedicle screw head (2).

6. The device of claim 5, wherein the coupling fittings are formed of two forks capable of being connected in jointed fashion, to enable to space apart the parts (1a) and (1b) to position them with respect to the pedicle screw head (2), and then to push them back in order to clamp said head maintained between said parts by holding means.

7. The device of any of claims 1, 5 and 6, **characterized in that** the other end of each part (1a) and (1b) cooperates, after having been pushed back, with a hollow connection nut (6) to form the tubular element as such, said parts (1a) and (1b) delimiting the diametrically opposite slots (1c) and (1d).

8. The device of claim 1, **characterized in that** a handling rod of variable length connects a group of tubular elements selected after correction and alignment in the three planes of space, to ensure a selective mobilization enabling to correct a gibbosity.
